# EUROPEAN PATENT APPLICATION

(11) **EP 4 088 822 A1**
(43) Date of publication of application: **16.11.2022**
(21) Application number: 21173528.7
(22) Date of filing: 12.05.2021
(51) Int. Cl.: B05B 17/00, A61M 15/00, B23K 26/36

(54) **NEBULIZING DEVICE FOR AN INHALATOR**

(71) Applicant: PARI Pharma GmbH, 82319 Starnberg (DE)
(72) Inventor: Dr. Denis, Thomas, 82319 Starnberg (DE)
(74) Representative: Hoffmann Eitle

(57) **Abstract**

The disclosure relates to a nebulizing device (2) for an inhalator (100). The nebulizing device (2) comprises an aperture plate (6) having a fluid side (7) to which a fluid (110) is to be supplied and a nebulizing side (9) at which the fluid is to be nebulized, wherein the aperture plate (6) comprises a single layer (5) of unitary material, a plurality of recesses (10) being formed in the single layer (5), the recesses (10) each having an opening (11) facing the fluid side (7) and a bottom (12) facing the nebulizing side (9), a plurality of the apertures (20) being formed in the bottom (12) of each of the recesses (20).

## Description

### Technical Field

The present disclosure relates to a nebulizing device for an inhalator and particularly to an aperture plate thereof. The present disclosure also relates to a method for manufacturing of such an aperture plate.

### Background

EP 2 717 951 B1 describes a method for manufacturing an aperture plate from a single layer of unitary material, particularly stainless steel. In this method, a plurality of apertures, each penetrating the single layer, is formed by laser drilling. For example, three laser drilling stages are used to form each of the apertures, wherein the fluence of the laser decreases from the first laser drilling stage to the third laser drilling stage. In this context, the fluence is defined as the time-integrated flux of some radiation or particle stream. Specifically, the fluence F of a (one) laser pulse is the optical energy delivered per unit area. Its most common unit is J/cm². Due to this process, the cross-sectional area of each aperture decreases from a fluid side of the aperture plate to a nebulizing side of the aperture plate, thus forming tapered holes.

Even though this method works sufficiently well, some disadvantages are associated therewith.

To begin with, the known method embodies a short-pulse laser (such as a nanosecond laser). As a result, burrs are formed around the opening of the apertures at the fluid side of the aperture plate requiring a finishing step such as e. g. electro-polishing described in EP 1 429 888 B1. Such finishing step, however, tends to widen the opening of the apertures at the fluid side of the aperture plate and may even alter other parameters of the apertures, particularly those of the so-called "nozzle portion" formed in the last (e.g. third) stage of the laser drilling process. The widening of the openings and alteration of the parameters is difficult to control. Hence, the diameter of the opening may differ from one aperture to another aperture posing a problem on the reproducibility of the aperture plate.

In addition, in-process monitoring only allows limited conclusions on the actual characteristics of the apertures (length and diameter of each of the laser drilling stages).

In one example, as described in more detail in "Investigation of Optical Density for the Characterization of Nebulizer Meshes", Ross H.M. Hatley, Lucy E.A. Hardaker, Joseph Zarins-Tutt, Filippo Quadrelli, Brendan Hogan, Ronan Mac Loughlin, John N. Pritchard, Respiratory Drug Delivery 2016", light transmission inspection may be used. In light transmission inspection a light source is directed onto one side (e.g. fluid side) of the aperture plate and a CCD camera captures the light passing through the apertures on the opposite side (e.g. nebulizing side). Yet, the light transmission depends on the parameters of all three laser drilling stages so that no conclusion can be drawn on the actual characteristic (geometry including but not limited to length and diameter) of each of the laser drilling stages. More particular, one may not draw a conclusion on the particular geometry of the nozzle portion.

The characteristics of the apertures, particularly the diameter of an exit opening of the "nozzle portion", have a significant influence on the Mass Median Diameter (MMD in µm) and the Geometric Standard Deviation (GSD) of the aerosol produced by the inhalator and consequently on lung deposition. Additionally, the Total Output Rate (TOR in pg/min) of the inhalator is affected by the characteristics of the apertures, particularly the length and diameter of the nozzle portion, and as a result the duration of the therapy (single administration/inhalation regimen).

To conclude on the exact characteristics of the apertures, a polished cut image of the aperture plate had to be produced, thereby destroying the aperture plate. Subsequently the apertures were measured by use of a scanning electron microscope, which is time-consuming and costly. Thus, the method is unsuitable for an in-process control.

### Summary

Taking the aforesaid into account, it was an aim to provide a nebulizing device for an inhalator, particularly an aperture plate thereof, and a method for manufacturing such an aperture plate that can tackle at least one of the above disadvantages.

One aim was to provide a nebulizing device for an inhalator, particularly an aperture plate thereof, and a method for manufacturing such an aperture plate more easily enabling to conclude on, preferably measure, the characteristics of the apertures even more particularly those of the nozzle portion. In a preferred mode, this should be possible as in-process monitoring and/or without the need to destroy the aperture plate.

Another aim was to provide a nebulizing device for an inhalator, particularly an aperture plate thereof, and a method for manufacturing such an aperture plate improving manufacturing precision and/or ease of manufacture and/or reducing manufacturing costs.

Yet, advantages of the known nebulizing device for an inhalator, particularly the aperture plate thereof, relating to structural stability and/or its vibrational characteristics should be maintained.

One aspect of the present disclosure suggests a nebulizing device for an inhalator. The nebulizing device comprises an aperture plate having a fluid side to which a fluid is suppliable and a nebulizing side at which the fluid is nebulized, e.g. upon vibration of the aperture plate and/or the fluid.

The aperture plate comprises a single (one single) layer of unitary material. A single layer of unitary material has no distinct borders, boundary surfaces or confining layers or the like in a polished cut image of the single layer. The aperture plate may in one embodiment comprise further layers attached to the single layer. In other embodiments, the aperture plate may consist of the single layer.

A plurality of recesses is formed in the single layer. The recesses each have an opening facing the fluid side or - in case the aperture plate consists of the single layer - at the fluid side of the aperture plate. The recesses each have a bottom opposite to the opening, i.e. at a side facing the nebulizing side or at the nebulizing side of the aperture plate. The recesses may also be referred to as blind holes introduced from one side of the single layer (from the fluid side (first side) of the aperture plate). In one aspect, at least two recesses are provided. In another aspect, at least three recesses are provided. Further, the number of recesses is not limited but less than 3,000, less than 1,000 or less than 500 may be preferred. Alternatively, less than 50 or less than 20 recesses may be provided. A relatively large number of smaller recesses, such as between 500 and 3,000 or 500 and 1,000, may be beneficial to achieve a high stability of the aperture plate. The more recesses are provided in the aperture plate, the higher the remaining reinforcing portions between the recesses providing for the full thickness of the single material layer and, hence, stability. To the contrary, a smaller number of larger recesses, such as between 2 and 50 or 2 and 20, may be beneficial to achieve a larger effective area for nebulization with less dead areas. In this context, "dead areas" are those areas of the aperture plate having no penetrating apertures and, hence, do not participate in the nebulization process. To put it differently, in these areas no fluid may pass through the aperture plate for being nebulized at the fluid side. Additionally, the manufacturing process may be simplified when using a smaller number of recesses. The area of the recesses relative to the total area of the aperture plate does, in one example, not exceed 50%.

Further, a plurality of apertures is formed in the bottom of each of the recesses. The plurality of apertures may each have an entrance opening formed in the bottom of the respective recess and an exit opening facing or formed at the nebulizing side of the aperture plate. According to an aspect, there are no additional apertures formed in the aperture plate other than those formed in the bottom of the recesses. According to another aspect, there are at least no additional apertures formed in the single layer between recesses and being blind holes.

Thus, the fluid on the fluid side of the aperture plate passes, upon vibration of the aperture plate and/or the fluid, through the recesses and apertures and is nebulized while exiting the apertures via the exit openings at the nebulizing side of the aperture plate. Thus, an aerosol is formed at the nebulizing side of the aperture plate for inhalation by a patient.

Due to the openings of the recesses having a relatively large area, a relatively small aspect ratio is achievable. To be more precise, state of the art three-dimensional microscopes may only be used for inspecting the geometry under appropriate lighting conditions. Those conditions can be met, if the aspect ratio, i.e. depth of the recesses relative to smallest dimension of the opening of the recesses (e.g. the diameter when considering a circular recess), is sufficiently low (aspect ratio = depth/smallest dimension, e.g. diameter considering a circular recess). The aspect ratio may for example be equal to or less than 2, equal to or less than 1.5, equal to or less than 1 or equal to or less than 0.6.

Accordingly in-process inspection via the openings may be realized. Thus, the characteristics of the recesses as well as of the entrance openings of the apertures formed in the bottom of the recesses may be measured with state-of-the art three-dimensional microscopy without the need to destroy the aperture plate.

Due to the use of the single layer of unitary material, structural stability of the aperture plate as well as its vibrational characteristics may nevertheless be maintained.

Additionally, the entrance opening of the apertures can be relatively small so that the density of the apertures may be increased as compared to the density of the three-stage laser-drilled apertures of the prior art, thus enabling to increase the TOR.

Even further and as particularized above, the TOR and MMD are strongly dependent on the geometry of the nozzle portion, i.e. of the aperture geometry (particularly length and diameter). Due to the formation of the recesses, a small and relatively even or constant thickness of the bottom of the recesses is achieved. Accordingly, the length of the apertures is very well defined. In addition, the formation of relatively short apertures is much more precise regarding diameter at an entrance side (corresponding to the fluid side) and an exit side (corresponding to the nebulizing side). As a result, a much more consistent geometry of the apertures relative to each other may be achieved, reducing the GSD. Consequently, aperture plates may be manufactured with an improved reproducibility as regards the geometry of the apertures and, hence, TOR, MMD and GSD. Hence, the characteristics of the aerosol (nebulized fluid) are more consistent when comparing different aperture plates obtained from the same manufacturing process.

The recesses and apertures may be manufactured by means of an etching process also used in the semiconductor industry as for example disclosed in "A silicon-based MEMS vibrating mesh nebulizer for inhaled drug delivery", Oskar Z. Olszewskia, Ronan MacLoughlinb, Alan Blakea, Mike O'Neillc, Alan Mathewsona, Nathan Jackson, 30th Eurosensors Conference, EUROSENSORS 2016. Suitable methods are wet etching, plasma etching, reactive-ion etching, deep reactive ion-etching and the like.

Alternatively, electron-beam machining may be used, wherein electron beam drilling may be used for drilling the apertures and electron-beam milling may be used for forming the recesses. The electron-beam may also be substituted by an ion beam.

The recesses and/or apertures may also be formed by a mechanical punching process, such as the one described in "Micro-hole fabrication by mechanical punching process", Byung-Yun Joo, Sung-Han Rhim, Soo-Ik Oh, Journal of Materials Processing Technology 170 (2005) 593-601.

In one embodiment, however, the recesses are laser milled and/or the apertures are laser drilled. The use of a laser milling process for forming the recesses is also beneficial from the viewpoint of achieving a relatively even and constant remaining material thickness in the area of the bottom of the recesses. Thus, an even more precise formation of the apertures regarding diameter and length may be achieved with the above described benefits relating to reproducibility of constant aerosol characteristics.

In a specific example, an ultra-short-pulse laser is used for laser milling. An ultra-short-pulse laser is defined as a laser using laser pulses of less than 10 picoseconds. An ultra-short-pulse laser has the benefit of being more precise and even further improving the benefits relating to reproducibility of constant aerosol characteristics described above.

The apertures may still be formed by using a short-pulse laser (e.g. a nanosecond laser) or by using an ultra-short-pulse laser. A short-pulse laser is defined as a laser using laser pulses of greater than 10 picoseconds and smaller than 500 nanoseconds.

Even when using a laser for manufacturing the recesses and/or the apertures, the formation of burrs at the opening of the recesses and/or the entrance openings of the apertures may be minimized or even be avoided. This particularly applies when using an ultra-short-pulse laser for forming the recesses, being an athermal process. In any case, the usually required finishing step (e.g. polishing) may be abolished and the manufacturing process is simplified. Additionally, the risk of widening of the openings by the polishing process is avoided.

As previously indicated, it is beneficial to use an ultra-short-pulse laser. Thus, a surface roughness of surfaces (circumferential wall and/or bottom) of the recesses and/or apertures may be relatively low so that finishing steps including polishing are superfluous. Accordingly, the arithmetical mean roughness (Ra) of surfaces of the recesses and/or apertures is equal to or less than 2 µm. When dealing with the roughness profile, the arithmetical mean height is referred to as the arithmetic mean roughness Ra. The arithmetical mean height indicates the average of an absolute value along a sampling length.

The aperture plate may after the formation of the recesses and apertures be domed, particularly in the area containing the recesses. In an alternative aspect, the aperture plate may remain flat.

Experiments have shown that the TOR may be increased when arranging the apertures in correspondence with the oscillating amplitude/-s of the aperture plate upon vibration thereof. On the other hand, the positioning of the apertures and/or the recesses and their shape also influence the vibrational characteristics of the aperture plate and, thereby, the location of the oscillating amplitude/-s.

Taking the aforesaid into account, at least some of the recesses are according to an example arranged in an area of an oscillating amplitude of the aperture plate upon vibration thereof. Thus, the TOR may be increased without increasing the number of apertures thus maintaining or even increasing stability of the aperture plate.

In one aspect, the nebulizing device further comprises an annular piezoelectric element (vibrator) for vibrating the aperture plate. Thus, the aperture plate is excited annularly.

From this perspective, the recesses may be arranged in the single layer in a rotationally symmetric pattern in a view on the fluid side of the single layer or aperture plate. It has been proven advantageous to use a rotationally symmetric pattern to match the recesses with the vibrational amplitude/-s of the aperture plate, at the same time achieving the required stability of the aperture plate.

Further, the shape of the recesses in top view on their openings or a cross-section perpendicular to the direction from the fluid side to the nebulizing side of the aperture plate may be of any kind. Those shapes include but are not limited to a rectangular shape, a square shape, a trapezoidal shape, parallelogram shape, a triangular shape, a linear shape, a grid like shape, a circular shape, an annular shape, an annular sector shape, a circular sector shape, a circular segment shape or combinations thereof. The use of the appropriate shape and location of the recesses, stability and vibrational characteristics of the aperture plate may be adjusted as needed. Yet, shapes having only a few or no edges or corners are preferred to avoid the formation of cracks during use.

Advantageously, the recesses comprise at least one annular recess and/or at least one circular recess. Again, the use of annular and/or circular recesses has proven advantageous to match the recesses with the vibrational amplitude/-s of the aperture plate, at the same time achieving the required stability of the aperture plate additionally avoiding or minimizing the risk of crack formation during use.

As previously indicated, the formation of larger recesses and the subsequent formation of a plurality of smaller apertures in the bottom of the recesses provides for the advantage that the size of the entrance openings of the apertures is relatively small (as compared to the entrance opening of a three stage laser drilled aperture) and the apertures may be arranged closer to each other. Accordingly, the apertures in the bottom of the recesses may be arranged with a pitch between 5 µm and 50 µm. In this context, the pitch is measured from the center of one aperture to the center of an adjacent aperture. As a result, the TOR may be increased without the need to increase the size of the aperture plate.

In one aspect, the density of the apertures in the bottom of the recesses is between 200 and 40,000 per mm² or 400 and 20,000 per mm² or 1,000 and 10,000 per mm². As a result, a plurality of apertures may be formed on a relatively small area thereby enabling to minimize the size of the aperture plate still achieving a relatively high TOR.

The recesses have a circumferential side wall. The circumferential side wall extends between the opening facing the fluid side of the aperture plate and the bottom of the respective recess.

In an aspect, the circumferential side wall of the recesses is stepped towards the bottom.

Due to this configuration, negative effects that may occur at the edges/circumferential side wall of the recesses can be avoided. Additionally, such configuration has the benefit that the fluid is guided towards the bottom of the recess and, hence, towards the entrance openings of the apertures. This is due to a tapering of opposite (facing) circumferential side walls or opposite (facing) portions of a circumferential side wall. This benefit may particularly apply to cases of relatively small recesses, e.g. a diameter of 40 µm to 70 µm (see below) but depending on the characteristics of the fluid to be nebulized (e.g. viscosity) also be valid for larger recesses.
In one aspect, the circumferential side wall has an inclined shape so that opposite (facing) circumferential side walls or opposite (facing) portions of a circumferential side wall taper towards each other in the direction of the bottom of the recess. "Inclined" means inclined relative to a direction from the fluid side to the nebulizing side of the aperture plate.

From the viewpoint of the simplest configuration for enabling measuring of the characteristics of the recesses, a straight shape of the circumferential side wall may be preferred. In this context, straight means parallel to the direction from the fluid side to the nebulizing side of the aperture plate (perpendicular to the bottom of the recess).

Finally, the circumferential side wall may have a concave or convex shape, i.e. a curved shape, relative to a direction from the fluid side to the nebulizing side of the aperture plate. Thus, the stiffness and vibrational properties of the aperture plate may be optimized. Additionally, as described above, fluid guidance towards the bottom of the recess and, hence, the entrance openings of the apertures is improved.

Additionally, potential edges or corners may be avoided, thus minimizing the risk of crack formation during use.

In one aspect, a smallest dimension of the opening of the recesses facing the fluid side is between 40 µm and 500 µm, preferably 70 µm and 400 µm and most preferred between 90 µm and 300 µm. In case the shape of the opening is circular, the smallest dimension is the diameter. In case of an annular opening, the smallest dimension is the width of the ring. In case of a rectangular shape, the smallest dimension is that of the smaller edge (leg) and so forth. Selecting the smallest dimension in the above range enables a relatively small aspect ratio. To be more precise, state of the art three-dimensional microscopes may only be used for inspecting the geometry under appropriate lighting conditions. Those conditions can be met, if the aspect ratio, i.e. depth of the recesses relative to smallest dimension of the opening of the recesses (e.g. the diameter when considering a circular recess), is sufficiently low (aspect ratio = depth/smallest dimension, e.g. diameter considering a circular recess). The aspect ratio may for example be equal to or less than 2, equal to or less than 1.5, equal to or less than 1 or equal to or less than 0.6. Hence, visual inspection of the characteristics of the respective recess and/or the apertures formed in the respective recess (particularly their entrance openings) during manufacture is enabled. Thus, in-process monitoring/inspection may be enabled. In this context, the smallest dimension is selected to be sufficiently large for obtaining the relatively small aspect ratio. The above definition of the smallest dimension of the opening of the recesses, hence, enables in-process monitoring/measuring but is at the same time sufficiently small to maintain the required stability of the aperture plate.

The size of the exit openings of the apertures has a significant impact on the MMD. Depending on the kind of fluid to be nebulized, the MMD is linearly larger than the diameter of the exit openings and, thus, directly proportional.

Accordingly, the apertures may be circular having a diameter (diameter of the exit openings) facing the nebulizing side between 1 µm and 7 µm, preferably between 1.5 µm and 5 µm and most preferred between 1.5 µm and 3 µm.

A length (which may also be referred to as height or depth) of the apertures in the single layer may be between 3 µm and 50 µm. In another example, the length of the apertures in the single layer may be between 5 µm and 20 µm. In an even further example, the length of the apertures in the single layer may be between 10 µm and 20 µm. Other ranges may be 5 to 15 µm, 5 to 12 µm or 10 to 15 µm.

In an example, the single layer has a thickness in a direction from the fluid side to the nebulizing side. The single layer may have a unitary thickness, but it is also conceivable that the single layer has different thicknesses in different areas.

The recesses may have a depth (which may also be referred to as length or height) equal to or more than 50% of the thickness of the single layer. In another example, the depth of the recesses may be between 70% and 95% or between 80% and 95% of the thickness of the layer. If the thickness of the single layer varies locally depending on the area of the single layer, the percentage is related to the thickness of the area in which the recess is provided. In this context, the thickness of the single layer in the area in which the recess is provided is unitary. The recesses may all have the same depth or may have different depths.

As the length of the apertures (see above) is governed by the depth of the recesses, the length of the apertures is the same within one recess but may be different between recesses. If the recesses all have the same depth, also the length of the apertures is the same as long as the single layer has a unitary thickness.

Each recess may have, at least 2, at least 5, at least 20, at least 50 or at least 100 of the apertures formed in the bottom. At most each recess may have 5,000 or 10,000 of the apertures.

The unitary material may in general be any kind of material. The primary criteria for the material are durability, machinability (depending on the process for forming the recesses and/or apertures as particularized above) and/or biocompatibility. Conceivable materials may include but are not limited to ceramics, plastic materials, semiconductor materials and metals. However, biocompatible metals such as titanium or stainless steel having a high stiffness and elasticity for efficient vibrational excitation have proven advantageous. In one particular example, the unitary material of the single layer may, therefore, be metal, particularly stainless steel. In this context, stainless steel provides for a relatively high stiffness and has already been proven to meet the requirements for efficient vibrational excitation and biocompatibility. Yet, when forming the recesses and/or holes by a short-pulse laser using relatively high fluence, there is a risk of burr formation at an entry side of the laser into the unitary material. This, however, may be avoided by providing the recesses and only forming the apertures in the bottom of the recesses. The problem may even be further alleviated when using the above-mentioned ultra-short-pulse laser. Hence, any subsequent electro-polishing process may be omitted.

Independent of the above nebulizing device, the present disclosure also concerns a method for manufacturing an (e.g. the above-described) aperture plate of a (the) nebulizing device for an (the) inhalator. Accordingly, any of the aspects and examples described above and associated features may also be embodied in the method described in the following.

The method comprises the step of providing one single layer of unitary material having a first side and a second side opposite to the first side. The first side may face or even be the fluid side of the aperture plate and the second side may face or even be the nebulizing side of the aperture plate as described above.

The method further comprises the steps of forming a plurality of recesses in the first side of the single layer, the recesses each having an opening at the first side and a bottom facing the second side, and forming a plurality of apertures, the plurality of apertures extending from the bottom of the recesses to the second side and having an opening at the second side of the single layer.

Accordingly and as previously indicated with respect to the aperture plate above, easier and improved process-monitoring and control is achievable. Additionally, the geometric characteristics of the apertures and/or the recesses are highly reproducible and obtainable without the need to destroy the aperture plate.

The recesses and/or the apertures may be formed by removing material from the single layer. When removing material from the single layer rather than displacing material, higher flexibility regarding shape, location and preciseness of the recesses and/or apertures may be achieved.

Beside the different processes described above for forming the recesses and/or apertures, which may also be embodied in the method, the recesses may be laser milled and/or the apertures may be laser drilled. The use of laser technology is beneficial as laser machining allows easy reconfiguration by reprogramming incorporated laser scanners to obtain a different geometry (shape and/or location) of the recesses/apertures. Laser machining does not require any complex tools (e.g. masks, punch or the like) which otherwise have to be produced anew when changing the geometry. Also in processes in which the material is grown, such as electroforming, changes of the geometry are cumbersome.

In a specific example, an ultra-short-pulse laser is used for laser milling. The apertures may still be formed by using a short-pulse laser or may as well be produced using an ultra-short-pulse laser. Even when using a laser for manufacturing the recesses and/or the apertures, the formation of burrs at the opening of the recesses and/or the entrance openings of the apertures may be minimized or even be avoided. This particularly applies when using the ultra-short-pulse laser for forming the recesses, being an athermal process. In any case, the usually required finishing step (e.g. polishing) may be abolished and the manufacturing process is simplified. Additionally, the risk of widening of the openings by the polishing process is avoided. As previously mentioned, an ultra-short-pulse laser may be used for laser milling the recesses and/or laser drilling the apertures. Additionally, if stainless steel is used as the unitary material, the formation of burrs at the first side and/or the bottom and/or the second side (entrance side of the laser beam) may be avoided. Hence, the step of electro-polishing may be abolished.

The apertures may be laser drilled with the same fluence. Hence, only one laser stage is necessary for forming the apertures in the bottom of the recesses. As the apertures only have to penetrate the bottom of the recesses (remaining thickness of the single layer), a relatively low fluence is sufficient for forming the apertures. Hence, the formation of burrs can effectively be avoided. Alternatively, the apertures in the bottom of the recesses may also be drilled with two laser stages having a different fluence. For example a first laser drilling stage with a first fluence may be used to form a first part of the aperture in the bottom of the recess and a second laser drilling stage with a second fluence lower than the first fluence may be used to finalize the aperture, i.e. completely penetrate the bottom and, hence, form the nozzle portion.

The recesses have a circumferential side wall. The circumferential side wall extends between the opening facing the fluid side of the aperture plate and the bottom of the respective recess. In an aspect, the circumferential side wall of the recesses is stepped towards the bottom.

Due to this configuration, negative effects that may occur at the edges/circumferential side wall of the recesses can be avoided. Additionally, such configuration has the benefit that the fluid is guided towards the bottom of the recess and, hence, towards the entrance openings of the apertures. This is due to a tapering of opposite (facing) circumferential side walls or opposite (facing) portions of a circumferential side wall. This benefit may particularly apply to cases of relatively small recesses, e.g. a diameter of 40 µm to 70 µm (see below) but depending on the characteristics of the fluid to be nebulized (e.g. viscosity) also be valid for larger recesses.

This advantage may as well be achieved when the circumferential side wall has an inclined shape so that opposite (facing) circumferential side walls or opposite (facing) portions of a circumferential side wall taper towards each other in the direction of the bottom of the recess. "Inclined" means inclined relative to a direction from the fluid side to the nebulizing side of the aperture plate.

From the viewpoint of the simplest configuration for enabling measuring of the characteristics of the recesses, a straight shape of the circumferential side wall may be preferred. In this context, straight means parallel to the direction from the fluid side to the nebulizing side of the aperture plate.

The aperture plate may after the formation of the recesses and apertures be domed particularly in the area containing the recesses. In an alternative aspect, the aperture plate may remain flat.

In one aspect, a smallest dimension of the opening of the recesses facing the fluid side is between 40 µm and 500 µm, preferably 70 µm and 400 µm and most preferred between 90 µm and 300 µm. In case the shape of the opening is circular, the smallest dimension is the diameter. In case of an annular opening, the smallest dimension is the width of the ring. In case of a rectangular shape, the smallest dimension is that of the smaller edge (leg) and so forth. Selecting the smallest dimension in the above range enables a relatively small aspect ratio. To be more precise, state of the art three-dimensional microscopes may only be used for inspecting the geometry under appropriate lighting conditions. Those conditions can be met, if the aspect ratio, i.e. depth of the recesses relative to smallest dimension of the opening of the recesses (e.g. the diameter when considering a circular recess), is sufficiently low (aspect ratio = depth/smallest dimension, e.g. diameter considering a circular recess). The aspect ratio may for example be equal to or less than 2, equal to or less than 1.5, equal to or less than 1 or equal to or less than 0.6. The above definition of the smallest dimension of the opening of the recesses, hence, allows visual inspection of the characteristics of the respective recess and/or the apertures formed in the respective recess (particularly their entrance openings) during manufacture. Hence, in-process monitoring/inspection may be enabled. In this context, the smallest dimension is selected to be sufficiently large for obtaining the relatively small aspect ratio. The above definition of the smallest dimension of the opening of the recesses, hence, enables in-process monitoring/measuring but is at the same time sufficiently small to maintain the required stability of the aperture plate.

The formation of larger recesses and the subsequent formation of a plurality of smaller apertures in the bottom of the recesses provides for the advantage that the size of the entrance openings of the apertures is relatively small and the apertures may be arranged closer to each other without overlap. Accordingly, the apertures in the bottom of the recesses may be arranged with a pitch between 5 µm and 50 µm. In this context, the pitch is measured from the center of one aperture to the center of an adjacent aperture. As a result, the TOR may be increased without the need to increase the size of the aperture plate.

The recesses may have a depth (which may also be referred to as length or height) equal to or more than 50% of the thickness of the layer. In another example, the depth of the recesses may be between 70% and 95% or between 80% and 95% of the thickness of the layer. If the thickness of the single layer varies depending on the area of the single layer, the percentage is related to the thickness of the area in which the recess is provided. In this context, the thickness of the single layer in the area in which the recess is provided is unitary. The recesses may all have the same depth or may have different depths.

As the length of the apertures (see above) is governed by the depth of the recesses, the length of the apertures is the same within one recess but may be different between recesses. If the recesses all have the same depth, also the length of the apertures is the same as long as the single layer has a unitary thickness. In particular, the recesses have been adapted so that the remaining thickness of the single layer corresponding to the bottom is still thick enough to maintain stability and stable vibrational characteristics but thin enough to allow one stage laser drilling at a constant fluence to finally drill the aperture. Only one drilling stage for forming the apertures is preferred to increase preciseness of the geometry of the apertures, i.e. the nozzle portion. As mentioned above, the TOR and MMD are strongly dependent on the geometry of the nozzle portion, i.e. of the aperture geometry (particularly length and diameter). The formation of relatively short apertures is much more precise regarding diameter at an entrance side (corresponding to the fluid side) and an exit side (corresponding to the nebulizing side). This particularly applies when using just one laser drilling stage (a plurality of laser pulses at a constant laser fluence) for forming each of the apertures in the bottom of the recess. As a result, a much more consistent geometry of the apertures relative to each other may be achieved. Consequently, aperture plates may be manufactured with an improved reproducibility as regards the geometry of the apertures and, hence, TOR, MMD and GSD. Hence the characteristics of the aerosol (nebulized fluid) are more consistent between different aperture plates obtained from the same manufacturing process.

In another aspect, the apertures in the bottom of the recesses may also be drilled with two laser drilling stages having a different fluence. For example a first laser drilling stage with a first fluence may be used to form a first part of the aperture in the bottom of the recess and a second laser drilling stage with a second fluence lower than the first fluence may be used to finalize the aperture, i.e. completely penetrate the bottom. In this instance, the nozzle portion may be defined by the second laser drilling stage only. Thus, the bottom may remain thicker (lower depth of the recess) when using more than one laser drilling stage for forming the apertures. This may provide for more mechanical rigidity of the aperture plate.

The unitary material may in general be any kind of material. The primary criteria for the material are durability, machinability (depending on the process for forming the recesses and/or apertures as particularized above) and/or biocompatibility. Conceivable materials may include but are not limited to ceramics, plastic materials, semiconductor materials and metals. However, biocompatible metals such as titanium or stainless steel having a high stiffness and elasticity for efficient vibrational excitation have proven advantageous. In one example, the unitary material of the single layer may, therefore, be metal, particularly stainless steel. In this context, stainless steel provides for a relatively high stiffness and has already been proven to meet the requirements for efficient vibrational excitation and biocompatibility. Yet, when forming the recesses and/or holes by a short-pulse laser using relatively high fluence, there is a risk of burr formation at an entry side of the laser into the unitary material. This, however, may be avoided by providing the recesses. Due to the recesses, the remaining material thickness of the single layer (thickness of the bottom of the recesses) is relatively small. Hence, the apertures may be formed in the bottom of the recesses using a relatively low fluence. Hence, the risk of burr formation at an entry side of the laser into the unitary material is reduced. The problem may even be further alleviated when using the above-mentioned ultra-short-pulse laser. Hence, any subsequent electro-polishing process may be omitted.

### Brief Description of the Drawings

In the drawings:
- Figure 1: shows a longitudinal cross-sectional view of an inhalator comprising a nebulizing device of the present disclosure.
- Figure 2: shows a plan view on the fluid side of the aperture plate of the nebulizing device shown in Figure 1 before doming.
- Figure 3: shows a cross-sectional view along the line 3-3 in Figure 2.
- Figure 4: shows an enlarged portion of the cross-sectional view of Figure 3.
- Figure 5A to I: show a plan view on the fluid side of an aperture plate indicating different geometries of the recesses.
- Figure 6A to G: show a cross-sectional view of different configurations of the circumferential side wall of the recesses.
- Figure 7: shows the vibration of an aperture plate during one vibrational period.

### Detailed Description

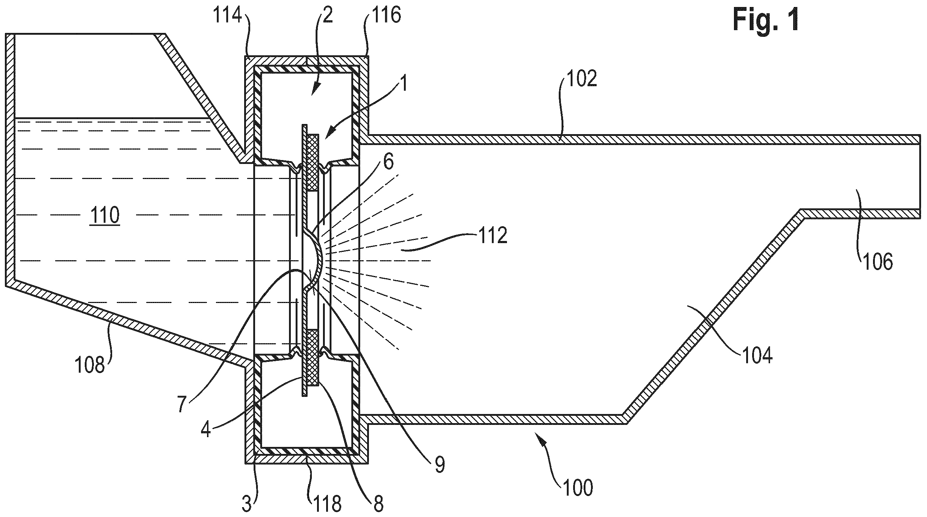

Figure 1 shows a schematic longitudinally cut cross-sectional view of an inhalator 100 comprising a nebulizing device 2.

The inhalator 100 comprises a first housing part 102 and a second housing part 108 which are joined to each other at a connection portion 118. The first housing part 102 has an aerosol chamber 104 and a mouthpiece 106. The second housing portion 108 forms a fluid chamber for receiving a fluid 110 to be aerosolized/nebulized.

A nebulizing device 2 is received in recesses 114, 116 formed in the second housing part 108 and the first housing part 102, respectively. The aerosol generator 2 comprises an annular housing 3 and a vibratable head 1 which is partially accommodated in the housing 3. The vibratable head 1 comprises an annular support member 4 and a circular vibratable membrane (aperture plate) 6 supported by the support member 4. The aperture plate 6 may be integrally formed with the support member 4 or be manufactured separately and be attached to the support member 4. The aperture plate 6 and the support member 4 are made from a metal, such as stainless steel. The aperture plate 6 has a plurality of recesses and apertures not visible in Figure 1 as will be particularized below.

The vibratable head 1 further comprises a vibrator 8 which is configured to vibrate the aperture plate 6. The vibrator 8 is attached by an adhesive to a surface portion of the support member 4. The vibrator 8 is an annular piezoelectric element made of ceramic.

In the following, operation of the inhalator 100 for the generation and delivery of an aerosol will be described.

A fluid 110 to be aerosolized/nebulized, for example, a fluid comprising an active compound, such as a drug substance or a medicament, is filled into the fluid reservoir formed by the second housing part 108.

The fluid 110 received in the fluid reservoir abuts at a fluid side 7 of the aperture plate 6 of the vibratable head 1.

A control (not shown) is operated to supply an activation signal to the vibrator 8 via electrical contacts (not shown) of the vibrator 8, activating the vibrator 8 and thus causing the aperture plate 6 to vibrate. The electrical contacts of the vibrator 8 may be provided in the form of one or more conductors, e.g., one or more flexible strip conductors, for example, printed circuit board tracks or strip lines. The one or more conductors may be attached to one or more surface portions of the vibrator 8, e.g., by an adhesive.

The fluid 110 abutting at the fluid side 7 of the aperture plate 6 is conveyed through the later described recesses and apertures in the aperture plate 6 and thereby aerosolized/nebulized into the aerosol chamber 104 at a nebulizing side 9 of the aperture plate 6. The aerosol 112 thus provided in the aerosol chamber 104 is inhaled by a patient or user through the mouthpiece 106 which is arranged in fluid communication with the aerosol chamber 104.

As visible from Figure 1, the aperture plate 6 may in the area of the recesses 10/apertures 20 be domed, i.e. convex towards the aerosol chamber 104 or concave towards the fluid 110.

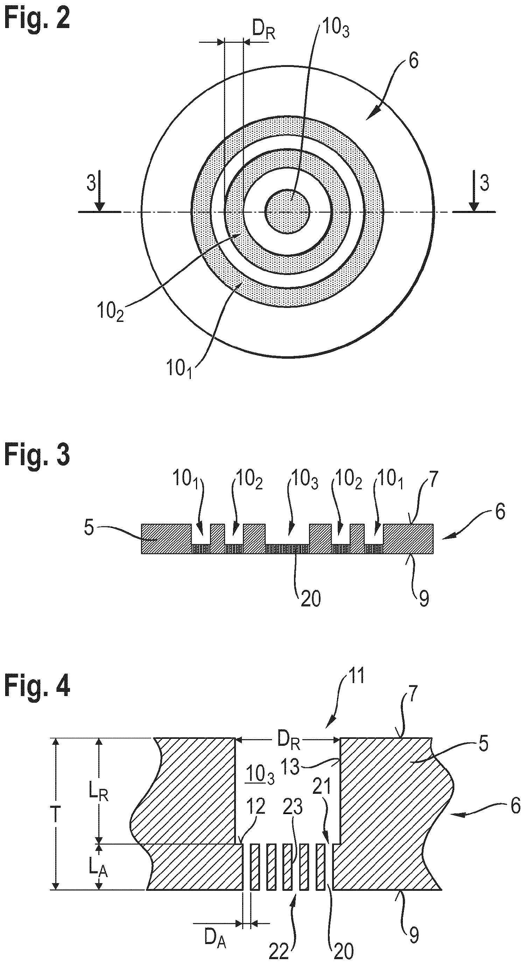

Figure 2 shows a plan view onto the aperture plate 6 as seen from the fluid side 7 thereof and before doming. Figure 3 shows a cross-sectional view along the line 3-3 in Figure 2.

In the example, the aperture plate 6 is circular and may be attached to the support member 4 or be integrally formed with therewith.

The aperture plate 6 in the example consists of a single layer 5 of stainless steel. Yet, in other embodiments, the aperture plate 6 may comprise further layers attached to the fluid side 7 (first side) and/or the nebulizing side 9 (second side). Additionally, other biocompatible metals may be used instead of stainless steel. Referring to the above summary section, also other materials may be used when manufacturing the single layer 5 of the aperture plate 6.

The exemplary aperture plate 6 comprises a plurality of recesses 10 (three in the example). The recesses 10 comprise two annular recesses 10₁ and 10₂ as well as one central circular recess 10₃. The annular recesses 10₁ and 10₂ are concentric to the circular recess 10₃ in the center. Thus, the recesses 10 are shaped and arranged so as to be rotationally symmetrical to the center of the aperture plate 6 coinciding with the center of the circular recess 10₃.

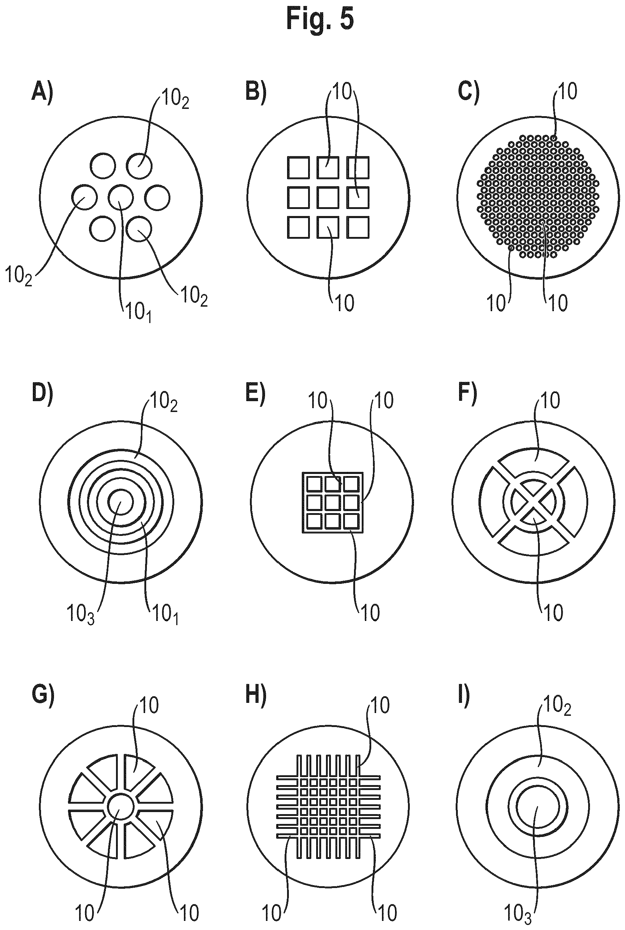

The recesses 10 have an opening 11 facing the fluid side 7 or in the example at the fluid side 7 of the aperture plate 6. The recesses are formed as blind holes having a bottom 12 opposite to the opening 11. The shape shown in the plan view in Figure 2 as well as in Figure 5 is governed by the opening 11 of the recesses.

Alternative geometries and arrangements of the recesses 20 are shown in Figures 5 A to I.

It becomes apparent that the shape of the recesses 20, i.e. of the openings 21 in plan view may vary. The shapes may include circular shapes (as in Figures 5A, 5C, 5D, 5G and 5I), annular shapes (as in Figures 5D and 5I), square shapes (as in Figure 5B), rectangular shapes (as in Figures 5B and 5E), line and grid shapes (as in Figures 5E and 5H), triangular shapes (as in Figure 5G), annular sections (as in Figure 5F), circular sections (as in Figure 5F), etc.. These shapes may be combined as desired. By choosing the shape and pattern of the recesses 20, the resonance frequency of the aperture plate 6 may be adjusted. Additionally, the eigenmode (natural frequency/resonance mode) of the vibration may be influenced by the selected pattern.

In the example, an annular piezoelectric element 8 is used for vibrating the aperture plate 6 so that the excitation of the aperture plate is rotationally symmetrical. As a consequence, it has been proven advantageous to use rotationally symmetric patterns, such as those shown in Figures 5A, C, D, F, G, I. It is to be noted that figure 5C is not perfectly rotational symmetric but still close enough to be considered as being rotational symmetric.

Even further, the structure should be divided in relatively small sections, thereby minimizing the risk of stress fracture. Moreover, edges should be avoided reducing the risk of notch stress. Finally, a central recess having a relatively large area has proven advantageous regarding stability during the doming process, i.e. curving/bending of the aperture plate 6 after the formation of the recesses 10 and the apertures 20.

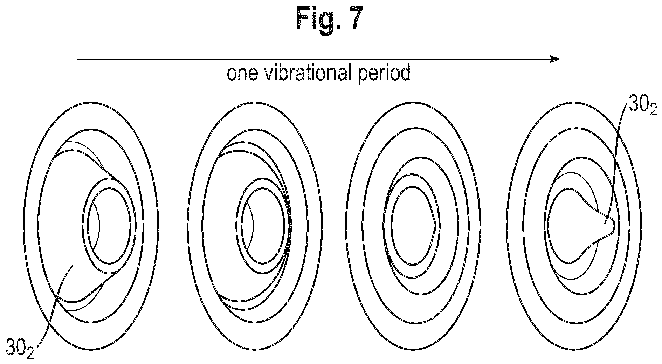

If the aperture plate 6 is excited by an annular piezoelectric element 8, the vibrational amplitude/-s during one vibrational period may be located in the center (30₁) and at a concentric ring (30₂) around the center (see Figure 7). Locating the or at least some of the recesses 10 in correspondence with the vibrational amplitude/-s may further increase the TOR without the need to provide a larger number of apertures 20.

Taking the aforesaid into account, the shapes and patterns as shown in Figures 5A, 5C, 5D and 5I promise the best results.

The pattern in Figure 5A comprises a plurality of equally sized circular recesses 10. A central recess 10₁ and a plurality of (six in the present example) recesses 10₂ arranged with their respective centers on a ring concentric to the central recess 10₁.

The pattern in Figure 5C comprises a plurality of recesses formed in a circular area preferably being equally distanced and arranged with their centers on concentric rings and/or radial lines.

The pattern in Figure 5D corresponds to the pattern shown in Figure 2. The pattern in Figure 5I differs from that in Figure 5D that only one annular recess 10 is provided rather than two as in Figure 5D.

The recesses 10 have a circumferential side wall 13. In case of the circular recess 10₃, the circumferential side wall 13 corresponds to the sheath of a cylinder. Yet, in the cross-section in Figures 3 and 4, portions of the circumferential side wall are opposite to (face) each other. In case of the circular recesses 10₁ and 10₂, two circumferential side walls 13 located opposite to each other or facing each other are provided.

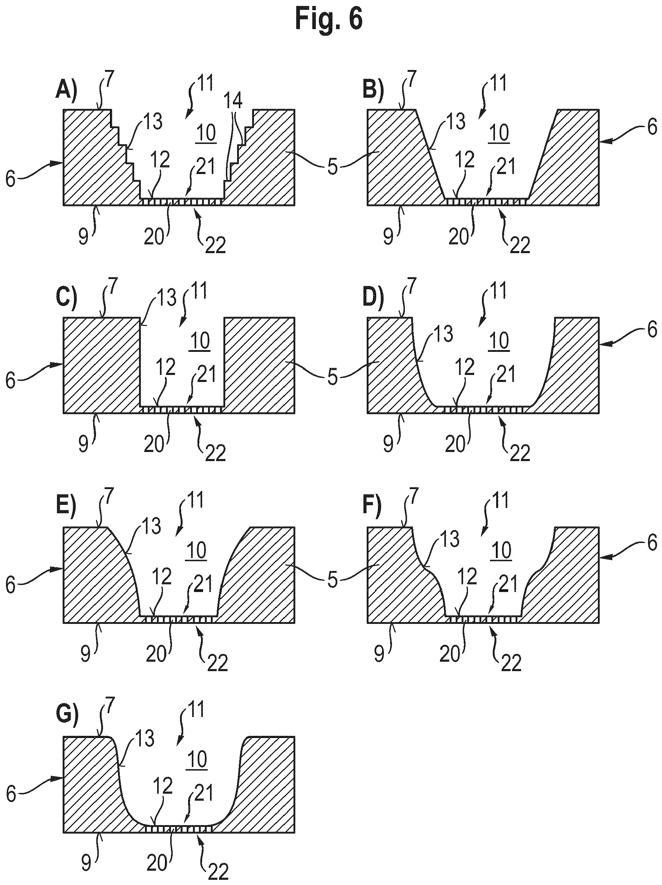

With reference to Figure 6, the circumferential side wall 13 may assume different shapes (geometries). As shown in Figures 6A and B, opposite portions of the circumferential side wall or facing circumferential side walls may taper towards the bottom 12. Thus, the fluid 110 may be guided towards the later described entrance openings 21 of the apertures 20.

In the example shown in Figure 6A, the circumferential side wall/-s is/are stepped having a plurality of steps 14. Such configuration has been proven advantageous when manufacturing the recesses by laser milling using an ultra-short-pulse laser and depending on the size of the recesses for guiding the fluid towards the entrance openings 21 of the apertures.

A similar effect may also be achieved when the circumferential side wall/-s 13 is/are is inclined relative to a direction from the fluid side 7 to the nebulizing side 9 as shown in Figure 6B. In this example, however, the circumferential side wall/-s 13 is/are straight rather than stepped.

The circumferential side wall/-s 13 may alternatively have a concave or convex shape, i.e. a curved shape, relative to a direction from the fluid side 7 to the nebulizing side 9 of the aperture plate. Thus, the fluid guidance towards or retention at the bottom 12 of the recess 10 and, hence, the entrance opening 21 of the apertures 20 is improved with respect to relatively small recesses with the smallest dimension of less than 70 µm. Additionally, potential edges or corners may be avoided, thus minimizing the risk of crack formation during use. A concave shape is shown in Figure 6D. A convex shape is shown in figure 6E and a funnel-shape is shown in Fig. 6F. The funnel shape may be beneficial to avoid stress peaks. Similar applies for the "S"-shaped circumferential side wall 13 in Fig. 6G.

From the viewpoint of the simplest configuration for enabling measuring of the characteristics of the recesses 10, a straight shape of the circumferential side wall/-s 13 may be preferred. In this context, straight means parallel to the direction from the fluid side 7 to the nebulizing side 9 of the aperture plate 6. Thus, opposite circumferential side walls 13 or opposite portions of one circumferential side wall 30 may extend parallel to each other. This configuration is shown in Figure 6C as well as in Figures 3 and 4.

As previously indicated, the recesses 10 may be formed in the single layer 5 by using an ultra-short-pulse laser. In this context, the recesses 10 are preferably made by laser milling, wherein the laser beam and/or the single layer 5 of the aperture plate 6 are translated relative to each other whereby material of the single layer 5 is gradually removed.

The smallest dimension D_{R} of the recesses 10 may be 300 µm for the central recess 10₃ and 250 µm for the annular recesses 10₁ and 10₂. However, other dimensions are as well conceivable. For example, a smallest dimension D_{R} of the opening 11 of the recesses 10 facing the fluid side 7 may be between 40 µm and 500 µm, between 70 µm and 400 µm or between 90 µm and 300 µm.

In this context, the smallest dimension D_{R} for the circular central recess 10₃ corresponds to the diameter of the circle. As regards the smallest dimension D_{R} of the annular recesses 10₁ and 10₂, the smallest dimension D_{R} corresponds to the width of the annular ring (see Figure 2).

The thickness T of the single layer 5 may be 100 µm.

The depth or length L_{R} of the recesses 10 may be selected between 80% to 95% of the thickness T of the single layer 5. Hence, the depth L_{R} of the recesses 10 may be between 80 µm to 95 µm.

However, the thickness T of the single layer 5 may reside in a range between 50pm to 200pm. Additionally, the depth L_{R} of the recesses 10 may be selected to be equal to or more than 50% of the thickness T of the single layer 5.

Each recess 10 has a plurality of apertures 20 formed in the bottom 12 and extending from the bottom 12 to the nebulizing side 9 (second side) of the single layer 5.

Each recess 10 may have at least 2, at least 20, or at least 50 or at least 100 of the apertures 20 formed in the bottom 12. At most each recess may have 5,000 or 10,000of the apertures 20.

Each aperture 20 has an entrance opening 21 at the bottom 12 and an exit opening 22 at the nebulizing side (second side) 9 of the single layer 5. The apertures 20 may be substantially cylindrical (straight side wall 23) or conical (inclined side wall 23) with the smaller opening being located at the nebulizing side 9 and thus being the exit opening 22. Yet, the geometry is not limited in this regard and other geometries are as well conceivable.

The size of the exit openings 22 of the apertures 20 has a significant impact on the MMD. Depending on the fluid, the MMD is linearly larger than the diameter D_{A} of the exit openings 22 and, thus, directly proportional. Accordingly, the apertures 20 may be circular having a diameter D_{A} (diameter of the exit openings) facing the nebulizing side between 1 µm and 7 µm, preferably between 1.5 µm and 5 µm and most preferred between 1.5 µm and 3 µm.

A length L_{A} (which may also be referred to as height or depth) of the apertures 20 in the single layer 5 may be between 3 µm and 50 µm. In another example, the length L_{A} of the apertures in the single layer 5 may be between 5 µm and 20 µm. In an even further example, the length L_{A} of the apertures in the single layer may be between 10 µm and 20 µm. Other ranges may be 5 to 15pm, 5 to 12pm or 10 to 15pm.

Even further and as particularized above, the TOR and MMD are strongly dependent on the geometry of the nozzle portion, i.e. of the aperture geometry (particularly length and diameter). Due to the formation of the recesses, a small and relatively even or constant thickness of the bottom of the recesses is achieved. Accordingly, the length of the apertures may be a very well defined. In addition, the formation of relatively short apertures is much more precise regarding diameter at an entrance side (corresponding to the fluid side) and an exit side (corresponding to the nebulizing side). As a result, a much more consistent geometry of the apertures relative to each other may be achieved, reducing the GSD. Consequently, aperture plates may be manufactured with an improved reproducibility as regards the geometry of the apertures and, hence, TOR, MMD and GSD. Hence, the characteristics of the aerosol (nebulized fluid) are more consistent between different aperture plates obtained from the same manufacturing process.

The apertures 20 may be formed by the same ultra-short-pulse laser used for forming the recesses 10. However, also a short-pulse laser, as used in the prior art, may be used for forming the apertures 20.

In any case, formation of the apertures 20 is preferably performed in one laser drilling stage at a substantially constant fluence of the laser. Only one drilling stage for forming the apertures is preferred to increase preciseness of the geometry of the apertures, i.e. the nozzle portion. When using just one laser drilling stage, the diameter at an entrance side (corresponding to the fluid side) and an exit side (corresponding to the nebulizing side) will be very well defined with the above described benefits.

In another aspect, the apertures in the bottom of the recesses may also be drilled with two laser drilling stages having a different fluence. For example a first laser drilling stage with a first fluence may be used to form a first part of the aperture in the bottom of the recess and a second laser drilling stage with a second fluence lower than the first fluence may be used to finalize the aperture, i.e. completely penetrate the bottom. In this instance, the nozzle portion may be defined by the second laser drilling stage only. Thus, the bottom may remain thicker (lower depth of the recess) when using more than one laser drilling stage for forming the apertures. This may provide for more mechanical rigidity of the aperture plate.

The method of manufacturing may include as a first step the formation of the recesses 10 from a first side (fluid side 7) of the single layer 5.

In a second, subsequent step, the apertures 20 are formed in the bottoms 12 of the recesses 10 from the first side of the single layer 5.

Subsequently and as previously mentioned, the aperture plate 6 and/or the single layer 5 may be domed.

### Reference List

- 1: vibratable head
- 2: nebulizing device
- 3: housing
- 4: support member
- 5: single layer
- 6: aperture plate
- 7: fluid side (first side)
- 8: vibrator
- 9: nebulizing side (second side)
- 10: recess
- 11: opening
- 12: bottom
- 13: circumferential side wall

- 20: aperture
- 21: entrance opening
- 22: exit opening
- 23: side wall

- 30: vibrational amplitude

- 100: inhalator
- 102: first housing part
- 104: aerosol chamber
- 106: mouthpiece
- 108: second housing part
- 110: fluid
- 112: aerosol
- 114: recess in the second housing part
- 116: recess in the first housing part
- 118: connection portion

## Claims

1. Nebulizing device (2) for an inhalator (100), the nebulizing device (2) comprising:
an aperture plate (6) having a fluid side (7) to which a fluid (110) is to be supplied and a nebulizing side (9) at which the fluid is to be nebulized,
wherein the aperture plate (6) comprises a single layer (5) of unitary material, a plurality of recesses (10) being formed in the single layer (5), the recesses (10) each having an opening (11) facing the fluid side (7) and a bottom (12) facing the nebulizing side (9), a plurality of the apertures (20) being formed in the bottom (12) of each of the recesses (20) .

2. Nebulizing device (2) according to claim 1, wherein the recesses (10) are laser milled and/or the apertures (20) are laser drilled.

3. Nebulizing device according to claim 1 or 2, wherein the arithmetical mean roughness (Ra) of surfaces of the recesses (10) is equal to or less than 2 µm.

4. Nebulizing device according to any one of the preceding claims, wherein at least some of the recesses (10) are arranged in an area of an oscillating amplitude (30) of the aperture plate (6) upon vibration thereof.

5. Nebulizing device according to any one of the preceding claims, further comprising an annular piezoelectric element (8) for vibrating the aperture plate (6), wherein the recesses (10) are arranged in the layer in a rotationally symmetric pattern.

6. Nebulizing device according to any one of the preceding claims, wherein the recesses (10) comprise at least one annular recess and/or at least one circular recess.

7. Nebulizing device according to any one of the preceding claims, wherein the apertures (20) in the bottom (12) of a recess (10) are arranged with a pitch between 5 µm and 50 µm.

8. Nebulizing device according to any one of the preceding claims, wherein the density of the apertures (20) in the bottom (12) of a recess (10) is between 200 and 40,000 per mm².

9. Nebulizing device according to any one of the preceding claims, wherein the recesses (10) have a circumferential side wall (13), the circumferential side wall (13) being stepped, inclined, curved or straight.

10. Nebulizing device according to any one of the preceding claims, wherein a smallest dimension (D_{R}) of the opening (11) of the recesses (10) facing the fluid side (7) is between 40 µm and 500 µm, preferably 70 µm and 400 µm and most preferred between 90 µm and 300 µm.

11. Nebulizing device according to any one of the preceding claims, wherein the apertures (20) are circular having a diameter (D_{A}) facing the nebulizing side between 1 µm and 7 µm, preferably between 1.5 µm and 5 µm and most preferred between 1.5 µm and 3 µm.

12. Nebulizing device according to any one of the preceding claims, wherein a length (L_{A}) of the apertures (20) in the single layer (5) is between 3 µm and 50 µm, preferably 5 µm and 20 µm and most preferred between 10 µm and 20 µm.

13. Nebulizing device according to any one of the preceding claims, wherein the single layer (5) has a thickness (T) in a direction from the fluid side (7) to the nebulizing side (9) and the recesses (10) have a depth (L_{R}) equal to or more than 50% of the thickness of the single layer (5), preferably between 80% and 95% of the thickness of the single layer (5).

14. Nebulizing device according to any one of the preceding claims, wherein each recess (10) has at least 2, or at least 20, or at least 50, or at least 100 of the apertures (20) formed in its bottom (12).

15. Nebulizing device according to any one of the preceding claims, wherein the unitary material of the single layer (5) is metal, preferably stainless steel.
